# EUROPÄISCHE PATENTSCHRIFT

(11) **EP 1 278 488 B1**
(45) Veröffentlichungstag und Bekanntmachung des Hinweises auf die Patenterteilung: **03.11.2004**
(21) Anmeldenummer: 01933734.4
(22) Anmeldetag: 21.03.2001
(51) Int. Cl.: A61F 2/46, B01F 13/00

(54) **AUFBEREITUNGS-UND APPLIKATIONSVORRICHTUNG FÜR ZU EINER PASTÖSEN FLIESSFÄHIGEN MASSE AUFZUBEREITENDE MATERIALEN, INSBESONDERE KNOCHENZEMENT**
PREPARATION AND APPLICATION DEVICE FOR MATERIALS TO BE PREPARED AS A PASTE-LIKE FLOWABLE MASS, ESPECIALLY BONE CEMENT
DISPOSITIF DE PREPARATION ET D'APPLICATION DE MATIERES DESTINEES A ETRE PREPAREES SOUS FORME DE MASSE FLUIDE PATEUSE, NOTAMMENT DE CIMENT D'OS

(30) Priorität: 05.05.2000 DE 20008103 U
(43) Veröffentlichungstag der Anmeldung: 29.01.2003
(73) Patentinhaber: Coripharm Medizinprodukte GmbH & Co. KG., 64807 Dieburg (DE)
(72) Erfinder: SATTIG, Christoph, 64807 Dieburg (DE); WÜST, Edgar, 63110 Rodgau (DE); WAHLIG, Helmut, 64287 Darmstadt (DE); DINGELDEIN, Elvira, 63303 Dreieich (DE)
(74) Vertreter: Helber, Friedrich G., Dipl.-Ing.
(86) Internationale Anmeldenummer: PCT/EP2001/003236
(87) Internationale Veröffentlichungsnummer: WO 2001/085070

(56) Entgegenhaltungen:
- EP-A- 0 380 867
- EP-A- 0 397 589
- EP-A- 0 768 067
- EP-A- 0 796 653
- EP-A- 0 824 895
- EP-A- 0 882 436
- WO-A-90/13264
- WO-A-97/18031
- DE-A- 4 302 230
- US-A- 4 277 184
- US-A- 4 961 647
- US-A- 5 435 645

## Beschreibung

Die Erfindung betrifft eine Aufbereitungs- und Applikationsvorrichtung für aus wenigstens einer pulver- oder granulatförmigen und einer flüssigen Komponente unmittelbar vor Gebrauch zu einer pastösen fließfähigen, zeitverzögert aushärtenden oder abbindenden Masse aufzubereitende Materialien, insbesondere Knochenzement, mit einem langgestreckten, zur Aufnahme der zu mischenden Komponenten vorgesehenen Mischzylinder, der in einem Endbereich mit einer verschließbaren und wahlweise einem Ausbringorgan für das aufbereitete pastöse Material zu versehenden Durchgangsöffnung und einem in das gegenüberliegende andere offene Ende einsetzbaren, im Zylinder verschieblichen und an der Zylinder-Innenwandung abdichtenden Kolben versehen ist, welcher von einem langgestreckten im Kolben abgedichteten Mischschaft verschieblich durchsetzt wird, an dessen zylinderinnerem Ende ein Mischorgan und an dessen zylinderäußeren Ende eine Handhabe angeordnet ist, wobei das der Durchgangsöffnung gegenüberliegende offene, den Kolben aufnehmende Ende des Zylinders zusätzlich mit einer auf dem Zylinder oder Kolben befestigbaren, von ihm abnehmbaren Verschlusskappe mit einer Durchtrittsöffnung für den Mischschaft versehen ist und der Kolben verschlusskappenseitig einen Sauganschluss zum Anschluss an einer Unterdruckquelle aufweist, welche über einen Durchlass im Kolben im Zylinderinnern ein Vakuum zu erzeugen vermag, wobei in der Verschlusskappe eine Durchgangsöffnung vorgesehen ist, durch welche die zur Unterdruckquelle führende Anschlussleitung hindurch am Sauganschluss im Kolben anschließbar ist.

Eine Vorrichtung gemäß dem Oberbegriff von Anspruch 1 ist aus der WO-A-97/18031 bekannt.

Knochenzement wird in der Regel aus einer pulverförmigen polymeren und einer flüssigen monomeren Komponente durch inniges Vermischen zu einer pastösen fließfähigen Masse aufbereitet, die dann nach der Einbringung beispielsweise in den Markkanal des Femur eines Patienten zur Befestigung des Schafts einer Hüftgelenkprothese aushärtet bzw. abbindet und so die dauerhafte belastbare Festlegung der Prothese im Femur gewährleistet. Bereits seit einer ganzen Reihe von Jahren ist es bekannt, dass die mechanische Festigkeit von Knochenzementen durch größere und kleinere Lufteinschlüsse, die insbesondere bei der Vermischung der Zementkomponenten in die resultierende Zementmatrix gelangen, erheblich reduziert wird. Die im Zement eingeschlossenen Luftblasen erzeugen Poren, die bei späterer Belastung durch die Prothese zu Riss- und Spaltenbildung im Zement führen, was eine vorzeitige Zerrüttung des die Prothese umgebenden Zementmantels und einer Prothesenlockerung mit der Notwendigkeit der Prothesenentfernung führen kann. Experimentelle und klinische Studien haben gezeigt, dass annähernd luft- und damit porenfreie Zemente zu einer höheren fatigue-Resistenz und damit zu einer Verlängerung der Lebensdauer von Endoprothesen beitragen. Es wurden deshalb Anmischsysteme für Knochenzement entwickelt, bei denen der Mischvorgang im Vakuum erfolgt. So wurden Anmischgefäße entwickelt, bei denen der Behälter nach dem Einfüllen der Komponenten verschlossen wird, so dass während des Mischvorgangs keine Monomerdämpfe entweichen können. Bei anderen Systemen erfolgt der Mischvorgang nach der Beschickung des Mischgefäßes unter reduziertem atmosphärem Druck. Dabei werden üblicherweise durch die Anwendung einer Vakuumpumpe die Monomerdämpfe abgesaugt und in einem Kohlefilter gebunden. Durch die Reduzierung des Drucks werden außerdem Lufteinschlüsse bei der Mischung der Zementkomponenten erheblich reduziert und so die Festigkeit des Knochenzements und somit die Lebensdauer einer mit Knochenzement im Knochen verankerten Endoprothese wesentlich erhöht.

Der Erfindung liegt die Aufgabe zugrunde, eine Vorrichtung für die Aufbereitung, d.h. der innigen Vermischung der Komponenten von beispielsweise Knochenzement unter Vakuum zu schaffen, die vergleichsweise einfacher als die bisher entwickelten Systeme aufgebaut ist und es ermöglicht, fertig angemischten Knochenzement in noch fließfähigem pastösem Zustand im vorgesehenen Befestigungsbereich zu applizieren. Die Vakuumeinwirkung soll dabei nur während des Mischvorgangs der Knochenzementkomponente einwirken, während der eigentliche Applikationsvorgang mit der zuvor wieder belüfteten, d.h. unter Atmosphären-Druck stehenden Vorrichtung durchgeführt wird.

Ausgehend von einer Vorrichtung der eingangs erwähnten Art wird diese Aufgabe erfindungsgemaß durch die kennzeichnenden Merkmale von Anspruch 1 gelöst. Durch die Verrastung des Kolbens mit der Verschlusskappe wird sicher gestellt, dass der nach dem Einfüllen der zu mischenden Materialkomponenten zusammen mit der Verschlusskappe auf dem Zylinder montierte Kolben infolge der Verriegelung der Kappe und des Kolbens bei der Betätigung des Mischorgans durch die Verschiebung und gleichzeitige Drehung des Mischschafts in der oberen Endstellung festgelegt ist. Aufgrund des über den Sauganschluss von der Unterdruckquelle ausgeübten Vakuums erfolgt der Mischvorgang dabei in der geforderten Weise im Vakuum.

Das Verriegelungselement bzw. die Verriegelungselemente sind dabei in bevorzugter Weiterbildung der Erfindung jeweils als langgestreckte flache Verriegelungslasche(n) ausgebildet, an welcher bzw. welchen die Verschlusskappe verschwenkbar angelenkt ist. Wenn die Verschlusskappe und die Verriegelungslasche(n) als Kunststoff-Spritzgussteile ausgebildet sind, ist es dann möglich, die Verriegelungslasche(n) über ein Filmscharnier integral an der Verschlusskappe anzulenken. Bei der Erfindung sind dabei zwei parallel zueinander verlaufende, mit ihren Flachseiten in einer Ebene liegende Verriegelungslaschen vorgesehen.

Die Verriegelungslaschen sind bei der Erfindung in jeweils einer den Kolben rechtwinklig zu dessen Längsmittelachse durchsetzenden, zum Querschnitt der Vierriegelungslaschen komplementären Durchgangsöffnung des Kolbens vormontiert, wobei im Rand des offenen Zylinders offen mündende Aussparungen mit verengter Mündung zur Aufnahme der Verriegelungslaschen vorgesehen sind. Dabei sind die Verriegelungslaschen dann in der bestimmungsgemäßen Verriegelungsstellung des Kolbens im offenen Ende des Zylinders liegenden Bereich in Querrichtung elastisch zusammendrückbar und so durch die verengte Mündung der Aussparung im Zylinder in die Aussparung einführbar. Durch die elastische Zusammendrückung in Querrichtung kann also der von den Verriegelungslaschen gehaltene Kolben zusammen mit der Verschlusskappe im offenen Ende befestigt werden.

Die elastische Zusammendrückbarkeit der Verriegelungslaschen in Querrichtung im Bereich der Aussparungen im Rand des Zylinders wird dabei mit Vorteil dadurch erreicht, dass in denen der bestimmungsgemäßen Verriegelungsstellung in den Aussparungen des Zylinders liegenden Bereichen mit langlochartigen Aussparungen versehen sind, welche eine Verformung der verbleibenden Randbereiche aufeinander zu ermöglichen.

Der Kolben, der den Kolben verschieblich durchsetzende, an seinem zylinderinneren Ende mit dem Mischorgan und am äußeren Ende mit der Handhabe versehene Mischschaft sowie die vom Mischschaft durchsetzte Verschlusskappe sind dabei zweckmäßig mit den in den Durchgangsöffnungen im Kolben vormontierten Verriegelungslaschen zu einer in das offene Ende des Zylinders einsetzbaren und mittels der Verriegelungslaschen in den im Rand des offenen Endes des Zylinders vorgesehenen Aussparungen verrastbaren Montage-Baugruppe vormontiert, die - nach dem Befüllen des Zylinders mit den Knochenzement-Komponenten - als Einheit in den Zylinder einführbar ist, wobei der Kolben und die Verschlusskappe im offenen Randbereich verrastet werden. Nach der Verrastung kann dann über die in der Verschlusskappe vorgesehene Durchgangsöffnung die Anschlussleitung zur Unterdruckquelle am Saugstutzen des Kolbens angeschlossen werden. Sobald dann das für die Mischung erforderliche Vakuum im Zylinderinnern erreicht ist, erfolgt die Vermischung der Komponenten durch gleichzeitiges Einschieben und Herausziehen sowie Drehen des Mischschafts, wodurch das Mischorgan die zu vermischenden Komponenten zum homogenen fließfähigen Knochenzement aufbereitet.

Nach der erfolgten Vermischung wird der Mischschaft so weit zurückgezogen, dass das Mischorgan an der zylinderinneren Stirnfläche des Kolbens anliegt und der Mischschaft wird dann in seinem außerhalb der Kappe liegenden Bereich abgebrochen.

Zweckmäßig ist der Mischschaft hierfür in dem bei ganz bis in Anlage an die zylinderinnere Stirnfläche des Kolbens verschobenen Lage des Mischorgans gerade im Bereich der zylinderabgewandten äußeren Stirnfläche des Kolbens stehenden Bereich mit einer Sollbruchstelle versehen, entlang derer der außerhalb des Kolbens stehenden Teil des Mischschafts abbrechbar ist. Aufgrund der verschwenkbaren Verbindung der Verschlusskappe mit den Verriegelungslaschen ist die Verschlusskappe dann hoch schwenkbar, wobei gleichzeitig zwangsläufig die Anschlussleitung zur Unterdruckquelle vom zugeordneten Saugstutzen im Kolben abgezogen und das Zylinderinnere belüftet wird. Nach dem Hochschwenken der Verschlusskappe können dann die Verriegelungslaschen aus den Aussparungen im Randbereich des Zylinders und den Durchgangsöffnungen im Kolben herausgezogen werden. Der Zylinder kann dann von Hand oder auch mittels des abgebrochenen Mischschafts in Abwärtsrichtung in Richtung auf das gegenüberliegende, noch durch einen eingeschraubten Verschlussstopfen verschlossene Ende geschoben werden, wodurch der Knochenzement zu einer kompakten Masse zusammengeschoben wird. In diesem Zustand wird der Zylinder dann in eine übliche Applikationspistole eingeführt und der Kolben dieser Applikationspistole bis in Anlage an die zylinderäußere Stirnfläche des Kolbens eingedrückt. Der bis dahin das Austreten von Knochenzement verhindernde Verschlussstopfen wird dann durch eine langgestreckte auch als "Schnorchel" bezeichnete Applikationsdüse ersetzt, so dass die plastische Knochenzementmasse durch Betätigung der Applikationspistole über den Schnorchel ausgepresst werden kann. Diese Applikationsdüse wird im Falle der Verwendung zur Befestigung des Schafts einer Endoprothese im Markkanal eines Knochens zweckmäßig so lang bemessen, dass sie bis unmittelbar vor dem vor der Einbringung des Schafts der Endoprothese im Markraum eingebrachten Markraumstopper geführt werden kann. Der Knochenzement wird dann beim Betätigen der Applikationspistole vom Markraumstopper aus in Richtung zum offenen Ende des Knochens steigend gefüllt, wobei noch im Befestigungsbereich des Schafts der Endoprothese befindliches Gewebewasser, Blut und andere Verunreinigungen sozusagen mit dem steigenden Spiegel des Knochenzements nach außen verdrängt werden und so nach dem Einführen des Schafts der Endoprothese in den mit Knochenzement gefüllten Markkanal in den bestehenden Zwischenräumen ausschließlich noch Knochenzement vorhanden ist.

Die das leichte Abbrechen des Schafts ermöglichende Sollbruchstelle wird zweckmäßig nicht in der Außenseite des Mischschafts erzeugt, da die hierfür erforderliche Einkerbung den am Mischschaft abdichtenden Dichtring beschädigen könnte.

In vorteilhafter Weiterbildung der Erfindung wird der Mischschaft deshalb von einem über seine gesamte Länge durchbohrten Röhrchen gebildet, wobei dann die Sollbruchstelle an einer in der Innenwandung des Röhrchens vorgesehenen umlaufenden eingekerbten Vertiefung gebildet wird.

Der Sauganschluss im Kolben wird zweckmäßig von einem Saugstutzen gebildet, dessen Saugkanal durch den Kolben hindurch in eine ringförmige umlaufende Vertiefung in der zylinderseitigen Stirnseite des Kolbens geführt ist, wobei die umlaufende ringförmige Vertiefung zum Zylinderinnern hin durch eine ringförmige poröse Scheibe aus einem Filtermaterial abgeschlossen ist, deren mittlere Porengröße so gewählt ist, dass sie zwar gasdurchlässig ist, Teilchen der in den Zylinder eingefüllten pulvrigen oder granulatförmigen Komponente oder auch den fertig aufbereiteten Knochenzement nicht hindurchtreten lässt.

Die ringförmige Scheibe aus Filtermaterial wird dabei zweckmäßig durch einen ihren äußeren Rand übergreifenden Clipring größeren Durchmessers und/oder einen ihren inneren Rand übergreifenden Clipring kleineren Durchmessers auf der zylindrischen Stirnfläche des Kolbens gehalten.

Dabei kann die Ausgestaltung mit Vorteil so getroffen sein, dass der Clipring größeren Durchmessers einen über einen gegenüber dem lichten Innendurchmesser des Zylinders im Durchmesser verkleinerten Umfangsabschnitt des Kolbens greifenden und/oder der Clipring kleineren Durchmessers einen in einen im Durchmesser gegenüber dem Durchmesser der im Kolben vorgesehenen Durchgangsbohrung für den Mischschaft im Durchmesser vergrößerten Bohrungsabschnitt eingreifenden Befestigungsabschnitt aufweist bzw. aufweisen.

Wenn dann der den im Durchmesser verringerten Umfangsabschnitt des Kolbens übergreifende Befestigungsabschnitt des Cliprings größeren Durchmessers und/oder der in den im Durchmesser vergrößerten Bohrungsabschnitt im Kolben eingreifende Befestigungsabschnitt des Cliprings kleineren Durchmessers in Richtung der Längsmittelachse des Kolbens jeweils kürzer als der zugeordnete Umfangs- bzw. Bohrungsabschnitt des Kolbens bemessen wird, ist es möglich, in den zwischen den Clipring-Befestigungsabschnitten und den im Kolben im Bereich der Durchmesseränderungen des Umfangs- bzw. Bohrungsabschnitts entstandenen Ringflächen gebildeten nutartigen Vertiefungen jeweils ein Dichtring angeordnet ist. Die Montage dieser - beispielsweise von O-Ringen gebildeten - Dichtringen ist dann vor dem Aufsetzen der Clipringe durch Aufschieben auf bzw. Einschieben in die Befestigungsabschnitte im Kolben sehr einfach montierbar.

Die Fixierung der die Scheibe aus Filtermaterial haltenden Clipringe am Kolben wird dann zweckmäßig dadurch gewährleistet, dass der den Kolben-Umfangsabschnitt über- und/oder der in den Bohrungsabschnitt eingreifende Befestigungsabschnitt des jeweiligen Cliprings auf- bzw. im zugeordneten Abschnitt des Kolbens verrastet und/oder verklebt und/oder durch Presssitz gehalten ist bzw. sind.

Die Erfindung ist in der folgenden Beschreibung eines Ausführungsbeispiels in Verbindung mit der Zeichnung näher erläutert, und zwar zeigt:
- Fig. 1: eine perspektivische Explosionsdarstellung einer erfindungsgemäßen Vorrichtung;
- Fig. 2: eine Seitenansicht der in Fig. 1 gezeigten Vorrichtung;
- Fig. 3: eine Schnittansicht der Vorrichtung, gesehen in Richtung der Pfeile 3-3 in Fig. 2;
- Fig. 4: eine Schnittansicht der Vorrichtung, gesehen in Richtung der Pfeile 4-4 in Fig. 3;
- Fig. 5: eine Draufsicht auf eine Stirnseite einer zum Auspresen der in der Vorrichtung aufbereiteten pastösen Masse in einem Mischzylinder verschieblich angeordneten Kolbens;
- Fig. 6: eine Schnittansicht des Kolbens in der durch die Pfeile 6-6 in Fig. 5 veranschaulichten Schnittebene;
- Fig. 7: eine Unteransicht auf ein im Mischzylinder vorgesehenes und am zylinderinneren Ende eines Mischschafts angeordneten Mischorgans;
- Fig. 8: eine Seitenansicht des Mischorgans, gesehen in Richtung des Pfeils 8 in Fig. 7;
- Fig. 9: eine Schnittansicht des Mischorgans, gesehen in Richtung der Pfeile 9-9 in Fig. 7;
- Fig. 10: eine perspektivische Ansicht einer Verschlusskappe, die gemeinsam mit dem Kolben nach dem Einbringen der zu vermischenden Komponenten in den Mischzylinder auf dessen oberem, offen mündenden Endbereich verrastbar ist;
- Fig. 11: eine Ansicht der Verschlusskappe, gesehen in Richtung des Pfeils 11 in Fig. 10; und
- Fig. 12: eine Schnittansicht durch die Verschlusskappe, gesehen in Richtung der Pfeile 12-12 in Fig. 11.

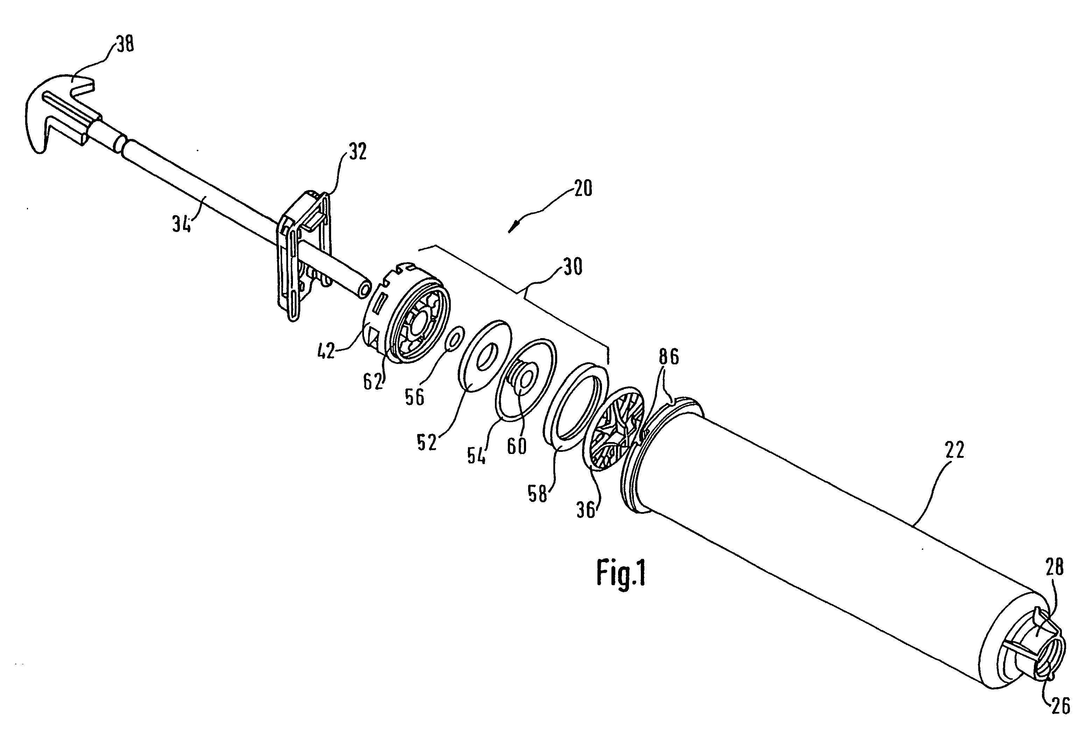

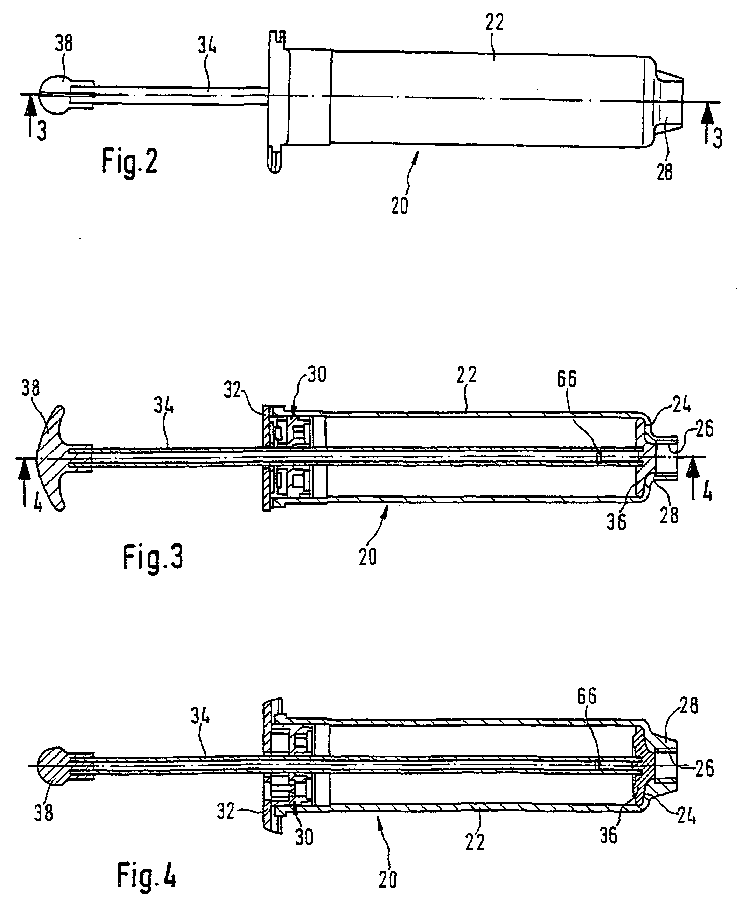

Die in den Fig. 1 in einer perspektivischen Explosionsdarstellung und den Fig. 2 bis 4 in einer Seitenansicht bzw. in zwei um 90° zueinander versetzten Ebenen dargestellte, in ihrer Gesamtheit mit 20 bezeichnete Vorrichtung dient zur Vermischung wenigstens einer pulverförmigen und einer flüssigen Komponente zu einer pastösen Masse, welche im speziellen Fall ein Knochenzement sein möge, der durch Vermischung einer pulverförmigen Polymer- und einer flüssigen Monomerkomponente innerhalb eines Mischzylinders 22 entsteht und nach dem Mischvorgang - entsprechend plastische Kleber- oder Dichtmassen enthaltenden Kartuschen - in ein (nicht gezeigtes) pistolenähnliches Applikationsgerät eingelegt werden kann, mittels dessen die im Mischzylinder aufbereitete Masse dann aus dem Zylinder 22 auspressbar und über eine dann am Zylinder angesetzte langgestreckte - auch als "Schnorchel" bezeichnete - Applikationsdüse in den vorgesehenen Anwendungsbereich, z.B. den Markkanal des Femur eines Patienten, eingebracht werden kann, in welchem der Schaft einer Hüftgelenk-Endoprothese mittels des Knochenzements fixiert werden soll.

Der aus Kunststoff hergestellte Mischzylinder 22 weist an seinem in den Zeichnungen rechts liegenden Ende eine Abschlusswand 24 mit einem von einer Gewindebohrung 26 durchsetzten vortretenden Stutzen 28 auf. Die Gewindebohrung 26 ist zunächst durch einen (nicht gezeigten) Gewindestopfen dicht verschlossen.

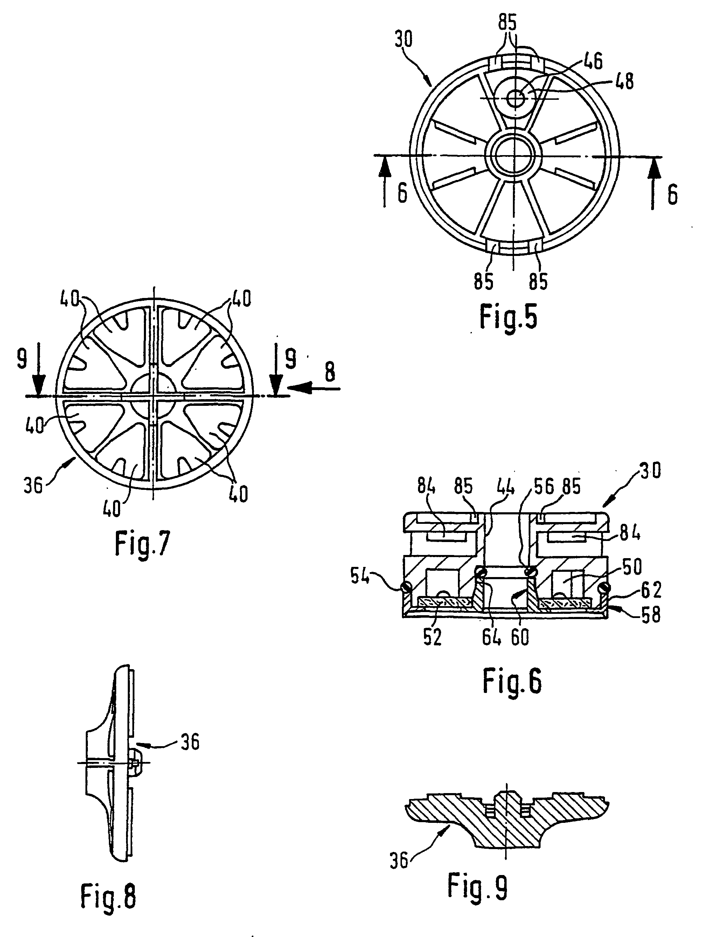

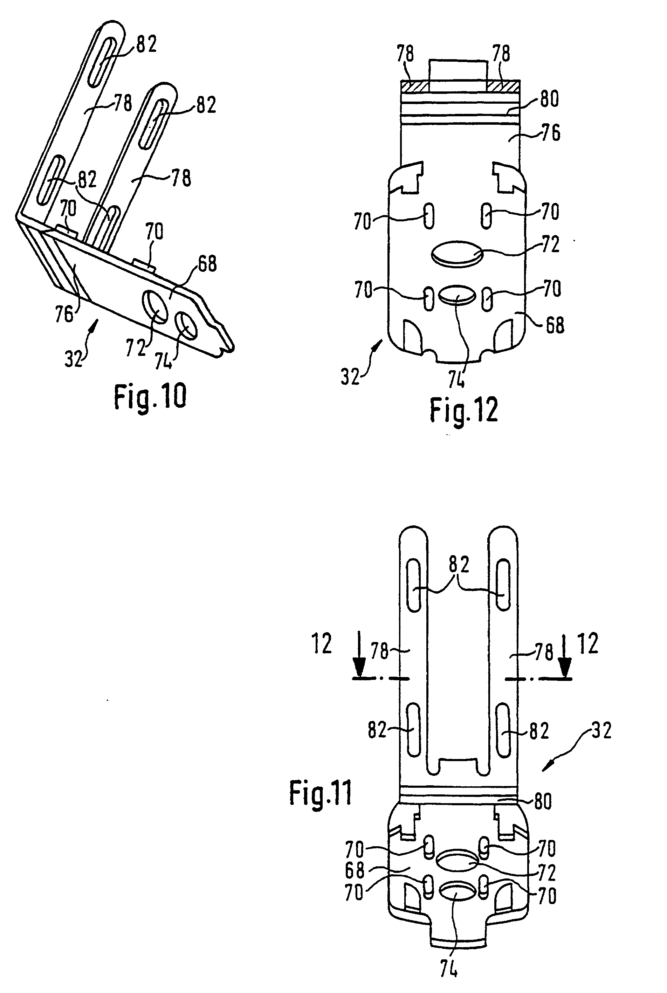

In das gegenüberliegende stirnseitig offen mündende Ende des Zylinders 22 sind die zu vermischenden Komponenten einfüllbar, worauf dann dieses Ende durch eine mit einem in den Zylinder einfüllbaren und in ihm verschiebbaren Kolben 30 (Fig. 5 und 6) verbundene Verschlusskappe 32 (Fig. 10 bis 12) auf dem offenen Ende verschliessbar ist.

Der Kolben 30 und die Verschlusskappe 32 werden von einem langgestreckten Mischschaft 34 längsverschieblich und verdrehbar durchsetzt, wobei am zylinderinneren Ende des als hohles Röhrchen ausgebildeten Mischschafts 34 ein auch als "Mischpaddel" bezeichnetes Mischorgan 36 und am gegenüberliegenden zylinderäußeren Ende eine Handhabe 38 befestigt sind. Speziell in den Fig. 3 und 4 ist erkennbar, dass das kreisförmig begrenzte und mit Durchbrechungen 40 versehene, am zylinderinneren Ende des Mischschafts 34 befestigte Mischorgan 40 innerhalb des Mischzylinders 22 über dessen gesamte Länge beweglich ist, und zwar durch Ausziehen und Einschieben des Mischschafts 34 durch eine an der Handhabe 38 angreifende Person. Auch eine Verdrehung des Mischschafts 34 mit dem an ihm befestigten Mischpaddel 36 ist durch entsprechende Drehung der Handhabe 38 möglich.

Der Kolben 30 ist aus den in Fig. 1 als Einzelteile dargestellten Bauteilen zusammengesetzt, nämlich dem eigentlichen zylindrisch begrenzten Kolbenkörper 42, dessen Außendurchmesser im wesentlichen gleich dem lichten Innendurchmesser des Mischzylinders 22 entspricht. Der Kolbenkörper 42 weist eine mittige Durchtrittsöffnung 44 auf, durch welche der Mischschaft 34 geführt ist. Radial versetzt zur Durchtrittsöffnung 44 wird der Kolben 30 noch von einem durchgehenden Saugkanal 46 durchsetzt, der auf seiner dem Zylinderinnern abgewandten Seite in einem im Kolben versenkt angeordneten Saugstutzen 48 mündet. Auf diesem Saugstutzen 48 ist beim Mischvorgang der in den Mischzylinder eingefüllten Komponente eine mit einer Unterdruckquelle, z.B. einer Vakuumpumpe verbundene Leitung befestigbar. Der Saugkanal 46 mündet nicht direkt in der zylinderinneren Stirnfläche des Kolbenkörpers, sondern in einer ringförmig umlaufenden Vertiefung 50, welche zum Zylinerinnern hin durch eine ringförmige poröse Scheibe 52 aus einem Filtermaterial abgeschlossen ist. Die Porösität dieser Filterscheibe 52 ist so gewählt, dass sie gasdurchlässig ist, d.h. im Zylinderinnern eingeschlossene Umgebungsatmosphäre sowie Monomer-Dämpfe der flüssigen Komponente durch die Scheibe hindurch zur Vakuumquelle absaugen kann, während pulver- oder granulatförmige Teilchen den Polymer-Komponenten im Zylinderinnern zurückgehalten werden. Auch die aufbereitete pastöse Masse kannn nicht durch die Filter-Scheibe 52 hindurchtreten.

Die Abdichtung des Kolbens im Zylinder 22 erfolgt durch einen äußeren, im speziellen Fall als O-Ring ausgebildeten Dichtring 54 und die Abdichtung des Mischschafts 34 in der Durchtrittsöffnung 44 durch einen inneren, ebenfalls als O-Ring ausgebildeten Dichtring 56, die durch jeweils einen äußeren bzw. inneren Clipring 58 bzw. 60 auf einen im Durchmesser entsprechend verringerten Umfangsabschnitt 62 des Kolbenkörpers 42 bzw. einem im Durchmesser entsprechend vergrößerten Bohrungsabschnitt 64 der Durchtrittsöffnung 44 im Kolbenkörper 42 gehalten sind. Gleichzeitig übergreifen die Clipringe 58, 60 die äußeren bzw. inneren Ränder der Filter-Scheibe 52 und fixieren diese so auf der zylinderinneren Stirnfläche des Kolbens 30.

In den Figuren 3 und 4 ist erkennbar, dass im zylinderinneren Ende des Mischschafts 34 mit Abstand vom Mischpaddel 36 eine Sollbruchstelle 66 in Form einer ringförmig umlaufenden Einkerbung in der Innenwandung des Mischschafts 34 vorgesehen ist. Der Abstand dieser Sollbruchstelle 66 vom Mischorgan 36 ist so bemessen, dass die Sollbruchstelle bei vollständig, d.h. bis in Anlage des Mischorgangs 36 an der zylinderinneren Stirnfläche des Kolbens 30 herausgezogenem Zustand die Sollbruchstelle gerade etwa mit der gegenüberliegenden, d.h. aus dem Zylinderinnern herausweisenden Stirnfläche des Kolbens 30 fluchtet. In diesem ganz herausgezogenen Zustand ist der Mischschaft 34 also relativ einfach durch Verbiegen an der Sollbruchstelle abbrechbar. Der verbleibende Teil des Mischschafts 34 und das Mischorgan 36 verbleiben dann - durch den inneren Dichtring 56 kleineren Durchmessers gehalten - in der am Kolben anliegenden Stellung des Mischorgangs.

Die Verschlusskappe 32 setzt sich aus dem eigentlichen Deckelteil 68 und in dem Kolben verrastbaren Keilen 70 zusammen. Die auf der Unterseite des Deckelteils befindlichen vier Verschlusskeile 70 werden in die dafür vorgesehenen, im Kolbenkörper 42 befindlichen Einschubnuten 85 eingerastet. In der Stirnwand des Deckelteils 68 ist mittig eine Durchtrittsöffnung 72 für den Mischschaft und radial zu ihr versetzt eine weitere Durchtrittsöffnung 74 vorgesehen, durch welche hindurch der Saugstutzen 48 im Kolben für den Anschluss einer mit der Unterdruckquelle verbundenen Leitung zugänglich ist. An einem radialen Ansatz 76 des Deckelteils 68 sind zwei parallele langgestreckte flache Verriegelungslaschen über ein Filmscharnier 80 integral angespritzt. In diesen Verriegelungslaschen 78 sind jeweils in der Nähe des Filmscharniers 80 und in der Nähe ihrer freien Enden langlochartige Aussparungen 82 vorgesehen, so dass die Verriegelungslaschen 78 im Bereich dieser Aussparungen 82 in Querrichtung elastisch zusammendrückbar sind.

Die Verschlusskappe 32 und der Kolben 30 können mittels der erwähnten Verriegelungslaschen 78 miteinander verbunden werden, indem die Verriegelungslaschen 78 in einer etwa parallel zum Deckelteil 68 verschwenkten Lage durch im Kolben 30 vorgesehene, den Kolben parallel zu seinen Stirnflächen durchsetzende Aussparungen 84 eingeschoben werden. Die mit den langlochartigen Aussparungen 82 versehenen Bereiche der Verriegelungslaschen 78 liegen dann zum Teil innerhalb der Aussparungen 84 des Kolbens und zum Teil in dem von der Umfangsfläche des Kolbens vortretenden Bereich der verriegelungslaschen 78.

Im Rand des offenen Endes des Zylinders sind offen mündende Aussparungen 86 mit verengter Mündung zur Aufnahme der Verriegelungslaschen 78 vorgesehen, in welche die Verriegelungslaschen 78 unter elastischer Zusammendrückung durch die langlochartigen Aussparungen 82 gebildeten Bereich einrastbar sind. Es ist also möglich, die gesamte vom Mischschaft 34 mit angesetztem Mischpaddel 36 und Handhabe 38, dem Kolben 30 und der mittels der Verriegelungslaschen 78 im Kolben vormontierten Verschlusskappe 32 gebildete Montage-Baugruppe nach dem Einfüllen der zu vermischenden Komponenten ins Innere des Mischzylinders 22 auf dem offenen Ende des Mischzylinders zu befestigen, indem die genannte Baugruppe mit dem Mischpaddel 36 vorausweisend in das offene Ende des Zylinders 22 eingeführt und dann der Kolben in den Zylinder eingeführt und bis zur Verrastung der Verriegelungslaschen 78 in die Rand-Aussparungen 86 des Zylinders 22 eingeschoben wird. Dabei verrasten die vier an der Unterseite des Deckelteils 68 befindlichen Verschlusskeile 70 in den dafür vorgesehenen, im Randbereich des Kolben befindlichen Einschubnuten 85, wodurch die Verschlusskappe in der bestimmungsgemäßen Befestigungsstellung auf dem Mischzylinder 22 gehalten ist. Durch die Aussparung 74 hindurch kann dann noch das freie Ende der an die Unterdruckquelle angeschlossenen Leitung auf den Saugstutzen 48 des Saugkanals 46 aufgesteckt und so das Innere des Zylinders evakuiert werden. Nach Erreichen des vorgesehenen Vakuums erfolgt dann die Vermischung des zuvor in den Mischzylinder 22 eingefüllten Komponenten durch Hin- und Herbewegung des an der Handhabe 38 ergriffenen Mischstabs 24 bis eine homogene Vermischung der beiden Komponenten erzielt ist.

Dann wird so verfahren, dass der Mischstab bis zur Anlage des Mischpaddels an der zylinderinneren Stirnseite des Kolbens hochgezogen wird, wobei durch Drehung des Mischpaddels noch ggf. an der Stirnfläche des Kolbens bzw. der dort vorgesehenen Filter-Scheibe 52 befindliche aufbereitete pastöse Masse abgestreift wird. In der ganz herausgezogenen Stellung des Mischschafts 24 wird dann der Mischschaft entlang der Sollbruchstelle 66 abgebrochen. Der Deckelteil 68 der Verschlusskappe 32 kann dann um das Filmscharnier 80 hochgeklappt werden, wobei gleichzeitig zwangsläufig das freie Ende der Saugleitung vom Saugstutzen 48 abgezogen und so das Innere des Mischzylinders belüftet wird. Auf diese Weise wird zwangsläufig erreicht, dass der Inhalt des Mischzylinders nur unter Atmosphärendruck - und keinesfalls unter verringertem Druck - zusammengeschoben und appliziert wird. Nach dem Hochschwenken des Deckelteils können dann die Verriegelungslaschen 78 aus den Ausnehmungen 84 im Kolben und den Aussparungen 86 im Rand des Mischzylinders 22 herausgezogen werden, so dass der Kolben dann frei verschieblich im oberen Ende des Mischzylinders liegt. Der Kolben wird dann - z.B. mit dem abgebrochenen Teil des Mischschafts - ins Zylinderinnere geschoben, wobei die aufbereitete pastöse Masse zu einem kompakten Klumpen zusammengeschoben und an die gegenüberliegende, mit der Gewindeöffnung 26 versehenen Stirnwand 24 des Mischzylinders 22 gedrängt wird. Der bis dahin in der Gewindebohrung 26 befindliche Verschlussstopfen kann dann gegen eine langgestreckte röhrchenförmige Applikationsdüse, den sog. "Schnorchel" ausgetauscht und der Mischzylinder in dieser Form in die übliche Applikationspistole eingelegt werden.

Der Vorschubkolben dieser Applikationspistole wird dann in Anlage an die äußere Stirnfläche des Zylinders geschoben und durch Betätigung des am Pistolengriff der Applikationspistole vorgesehenen Hebels ist der Vorschubkolben weiter in das Innere des Mischzylinders vorschiebbar, wobei der Kolben 30 dann die aufbereitete pastöse Masse in die Applikationsdüse und aus dieser heraus verdrängt. Auf diese Weise kann dann die aufbereitete Masse, d.h. im vorliegenden angenommenen Anwendungsfall der aufbereitete Knochenzement, mittels der Applikationsdüse gezielt im vorgesehenen Anwendungsbereich aufgebracht werden.

## Patentansprüche

1. Aufbereitungs- und Applikationsvorrichtung (20) für aus wenigstens einer pulver- oder granulatförmigen und einer flüssigen Komponente unmittelbar vor Gebrauch zu einer pastösen fließfähigen, zeitverzögert aushärtenden oder abbindenden Masse aufzubereitende Materialien, insbesondere Knochenzement, mit einem langgestreckten, zur Aufnahme der zu mischenden Komponenten vorgesehenen Mischzylinder (22) , der in einem Endbereich mit einer verschließbaren und wahlweise einem Ausbringorgan für das aufbereitete pastöse Material zu versehenden Durchgangsöffnung (26) und einem in das gegenüberliegende andere offene Ende einsetzbaren, im Zylinder (22) verschieblichen und an der Zylinder-Innenwandung abdichtenden Kolben (30) versehen ist, welcher von einem langgestreckten im Kolben (30) abgedichteten Mischschaft (34) verschieblich durchsetzt wird, an dessen zylinderinnerem Ende ein Mischorgan (36) und an dessen zylinderäußeren Ende eine Handhabe (38) angeordnet ist, wobei das der Durchgangsöffnung (26) gegenüberliegende offene, den Kolben (30) aufnehmende Ende des Zylinders (22) zusätzlich mit einer auf dem Zylinder oder Kolben befestigbaren, von ihm abnehmbaren Verschlusskappe (32) mit einer Durchtrittsöffnung (72) für den Mischschaft (34) versehen ist und der Kolben (30) verschlusskappenseitig einen Sauganschluss (48) zum Anschluss an einer Unterdruckquelle aufweist, welche über einen Durchlass im Kolben im Zylinderinnern ein Vakuum zu erzeugen vermag, wobei in der Verschlusskappe eine Durchgangsöffnung (74) vorgesehen ist, durch welche die zur Unterdruckquelle führende Anschlussleitung hindurch am Sauganschluss im Kolben (30) anschließbar ist,
**dadurch gekennzeichnet,**
**dass** die als über den Rand des offenen Endes des Zylinders (22) aufsetzbare Verschlusskappe (32) mit zwei flachen parallel zueinander verlaufenden, mit ihrer Flachseite in einer Ebene liegenden Verriegelungslaschen (78) beweglich verbunden ist, welche in jeweils einer den Kolben (30) rechtwinklig zu dessen Längsmittelachse durchsetzenden zum Querschnitt der Verriegelungslaschen (78) komplementären Durchgangsöffnung (84) des Kolbens (30) vormontiert sind,
**dass** im Rand des offenen Endes des Zylinders (22) offen mündende Aussparungen (86) mit verengter Mündung zur Aufnahme der Verriegelungslaschen (78) vorgesehen sind, und
**dass** die Verriegelungslaschen in den in der bestimmungsgemäßen Verriegelungsstellung des Kolbens (30) im offenen Ende des Zylinders liegenden Bereich in Querrichtung elastisch zusammendrückbar und so durch die verengte Mündung der Aussparungen (86) im Zylinder (22) in die Aussparungen (86) einführbar sind und die verschlusskappe (32) und den Zylinder (22) auf bzw. im offenen Endbereich des Zylinders (22) halten.

2. Vorrichtung nach Anspruch 1, **dadurch gekennzeichnet, dass** die Verriegelungslaschen (78) verschwenkbar an der Verschlusskappe (32) angelenkt sind.

3. Vorrichtung nach Anspruch 2, **dadurch gekennzeichnet, dass** die Verschlusskappe (32) und die Verriegelungslasche(n) Kunststoff-Spritzgussteile sind.

4. Vorrichtung nach Anspruch 3, **dadurch gekennzeichnet, dass** die Verriegelungslaschen (78) über ein Filmscharnier (80) integral an der Verschlusskappe (32) angelenkt ist bzw. sind.

5. Vorrichtung nach einem der Ansprüche 1 bis 4, **dadurch gekennzeichnet, dass** die Verriegelungslaschen (78) in den in der bestimmungsgemäßen Verriegelungsstellung in den Aussparungen (86) des Zylinders (22) liegenden Bereich mit langlochartigen Aussparungen (82) versehen sind, welche eine Verformung der verbleibenden Randbereiche aufeinander zu ermöglichen.

6. Vorrichtung nach einem der Ansprüche 1 bis 5, **dadurch gekennzeichnet, dass** der Kolben (30), der den Kolben (30) verschieblich durchsetzende an seinem zylinderinneren Ende mit dem Mischorgan (36) und am äußeren Ende mit der Handhabe (38) versehene Mischschaft (34) sowie die vom Mischschaft (34) durchsetzte Verschlusskappe (32) mit den in den Durchgangsöffnungen (84) im Kolben (30) vormontierten Verriegelungslaschen (78) zu einer in das offene Ende des Zylinders (22) einsetzbaren und mittels der Verriegelungslaschen (78) in den im Rand des offenen Endes des Zylinders (22) vorgesehenen Aussparungen (86) verrastbaren Montage-Baugruppe vormontiert sind.

7. Vorrichtung nach Anspruch 6, **dadurch gekennzeichnet, dass** der Mischschaft (34) in dem bei ganz bis in Anlage an die zylinderinnere Stirnfläche des Kolbens (30) verschobenen Lage des Mischorgans (36) gerade im Bereich der zylinderabgewandten äußeren Stirnfläche des Kolbens (30) stehenden Bereich mit einer Sollbruchstelle (66) versehen ist, entlang derer der außerhalb des Kolbens (30) stehende Teil des Mischschafts (34) abbrechbar ist.

8. Vorrichtung nach einem der Ansprüche 1 bis 7, **dadurch gekennzeichnet, dass** der Mischschaft (34) von einem über seine gesamte Länge durchbohrten Röhrchen gebildet wird, und dass die Sollbruchstelle (66) von einer in der Innenwandung des Röhrchens vorgesehenen umlaufenden eingekerbten Vertiefung gebildet wird.

9. Vorrichtung nach Anspruch 8, **dadurch gekennzeichnet, dass** der Sauganschluss im Kolben (30) von einem Saugstutzen (48) gebildet wird, dessen Saugkanal (46) durch den Kolben (30) hindurch in eine ringförmig umlaufende Vertiefung (50) in der zylinderseitigen Stirnseite des Kolbens (30) geführt ist, und dass die umlaufende ringförmige Vertiefung (50) zum Zylinderinnern hin durch eine ringförmige poröse Scheibe (52) aus einem Filtermaterial abgeschlossen ist, deren mittlere Porengröße so gewählt ist, dass sie gasdurchlässig ist, Teilchen der in den Zylinder (22) eingefüllten pulvrigen oder granulatförmigen Komponente und/oder die aus den Komponenten aufbereitete pastöse Masse jedoch nicht durchtreten läßt.

10. Vorrichtung nach Anspruch 9, **dadurch gekennzeichnet, dass** die ringförmige Scheibe (50) aus Filtermaterial durch einen ihren äußeren Rand übergreifenden Clipring (58) größeren Durchmessers und/oder einen ihren inneren Rand übergreifenden Clipring (60) kleineren Durchmessers auf der zylindrischen Stirnfläche des Kolbens (30) gehalten ist.

11. Vorrichtung nach Anspruch 10, **dadurch gekennzeichnet, dass** der Clipring (58) größeren Durchmessers einen über einen gegenüber dem lichten Innendurchmesser des Zylinders (22) im Durchmesser verkleinerten Umfangsabschnitt (62) des Kolbens (30) greifenden und/oder der Clipring (60) kleineren Durchmessers einen in einen im Durchmesser gegenüber dem Durchmesser der im Kolben (30) vorgesehenen Durchgangsbohrung (44) für den Mischschaft (34) im Durchmesser vergrößerten Bohrungsabschnitt (64) eingreifenden Befestigungsabschnitt aufweist bzw. aufweisen.

12. Vorrichtung nach Anspruch 10 oder 11, **dadurch gekennzeichnet, dass** der den im Durchmesser verringerten Umfangsabschnitt (62) des Kolbens übergreifende Befestigungsabschnitt des Cliprings (58) größeren Durchmessers und/oder der in den im Durchmesser vergrößerten Bohrungsabschnitt (64) im Kolben (30) eingreifende Befestigungsabschnitt des Cliprings (60) kleineren Durchmessers in Richtung der Längsmittelachse des Kolbens (30) jeweils kürzer als der zugeordnete Umfangs- bzw. Bohrungsabschnitt des Kolbens bemessen ist, und dass in den zwischen den Clipring-Befestigungsabschnitten und den im Kolben im Bereich der Durchmesseränderungen des Umfangs- bzw. Bohrungsabschnitts (62 bzw. 64) entstandenen Ringflächen gebildeten nutartigen Vertiefungen jeweils ein Dichtring (54 bzw. 56) angeordnet ist.

13. Vorrichtung nach Anspruch 11 oder 12, **dadurch gekennzeichnet, dass** der den Kolben-Umfangsabschnitt (62) über- und/oder der in den Bohrungsabschnitt (64) eingreifende Befestigungsabschnitt des jeweiligen Cliprings (58; 60) auf- bzw. im zugeordneten Abschnitt des Kolbens (30) verrastet und/oder verklebt und/oder durch Preßsitz gehalten ist bzw. sind.

## Claims

1. Preparation and application device (20) for materials which are to be prepared as a paste-like, flowable mass from at least one powder or granulate component and one liquid component, especially bone cement, with an elongated mixing cylinder (22) for receiving the components to be mixed, comprising in one end region a sealable through-opening (26) that can optionally be provided with a discharge unit for the paste-like material to be prepared, and a piston (30) which can be inserted into the other, opposite, open end, which piston is displaceable in the cylinder (22) and seals against the inner wall of the cylinder, the piston being penetrated by an elongated mixing shaft (34) sealed against the piston (30), with a mixing mechanism (36) disposed at the cylinder inside-end of the mixing shaft and a handle (38) disposed at the cylinder outside-end of the mixing shaft, wherein the open end of the cylinder (22) located opposite the through-opening (26) and receiving the piston (30) is additionally provided with a removable sealing cap (32) that can be attached to and removed from the cylinder or piston and has a through-opening (72) for the mixing shaft (34), wherein the piston (30) on the side of the sealing cap has a vacuum fitting (48) adapted for connection to a vacuum source, which vacuum source can produce a vacuum in the interior of the cylinder through a passageway provided in the piston, wherein the sealing cap includes a through-opening (74) through which through-opening the vacuum line connected to the vacuum source can be connected to the vacuum fitting in the piston (30),
**characterized in**
**that** the sealing cap (32), which can be placed over the edge of the open end of the cylinder (22), is movably connected to two mutually parallel flat locking brackets (78) having flat surfaces located in a plane and which are pre-mounted in corresponding through-openings (84) of the piston (30), which through-openings penetrate the piston (30) perpendicular to its longitudinal center axis and are complementary to the cross-section of the locking brackets (78),
**that** in an edge region of the open end of the cylinder (22) there are provided open-ended recesses (86) with a narrowed mouth for receiving the locking brackets (78), and
**that** the locking brackets (78) in the intended locking position of the piston (30) can be elastically pressed together in the transverse direction in the region located at the open end of the cylinder (22)) and thereby be inserted in the cylinder (22) through the narrowed mouth of the recesses (86) disposed in the cylinder (22) and openings (84) provided in the piston (30).

2. Device according to claim 1, **characterized in that** the locking element(s) is/are formed as elongated flat locking bracket(s) (78), with the sealing cap (32) pivotally joined with the locking bracket(s).

3. Device according to claim 2, **characterized in that** the sealing cap (32) and the locking bracket(s) (78) are injection molded plastic parts.

4. Device according to claim 3, **characterized in that** the locking bracket(s) (78) is/are integrally pivotally joined to the sealing cap (32) via a film hinge (80).

5. Device according to one of claims 1 to 4, **characterized in that** the locking brackets (78) are provided with elongated through-holes (82) disposed in a region that in the intended locking position is located in the recesses (86) of the cylinder (22), which through-holes (82) permit a relative deformation of the remaining edge regions.

6. Device according to one of the claims 1 to 5, **characterized in that** the piston (30), the mixing shaft (34) which movably penetrates the piston (30) and is provided on its cylinder-interior end with the mixing mechanism (36) and on the exterior end with a handle (38), as well as the sealing cap (32) that is penetrated by the mixing shaft (34), together with the latching brackets (78) that are pre-mounted in the through-openings (84) in the piston (30), are pre-mounted in the form of an assembly that can be inserted into the open end of the cylinder (22) and locked by engaging the locking brackets (78) in the recesses (86) disposed in the edge region of the open end of the cylinder (22).

7. Device according to claim 6, **characterized in that** the mixing shaft (34), when the mixing mechanism (36) is moved completely into a position so as to contact the cylinder-internal end face of the piston (30), is provided in the region of the outer end face of the piston (30) with a rated break point (66), along which the portion of the mixing shaft (34) positioned outside the piston (30) can be broken off.

8. Device according to one of claim 1 to 7, **characterized in that** the mixing shaft (34) is formed by a small tube having a through-bore extending along its entire length, and that the rated break point (66) is formed by a circumferentially notched indentation disposed in the interior wall of the small tube.

9. Device according to one of the claims 1 to 8, **characterized in that** the vacuum fitting in the piston (30) is formed by a vacuum nipple (48) having a vacuum channel (46) which is guided through the piston (30) into a ring-shaped circumferential recess (50) in the cylinder-side end face of the piston (30), and that the ring-shaped circumferential recess (50) is closed off to the inside of the cylinder by a circular porous disk (52) made of a filter material, wherein the average pore size of the filter material is selected so as to be permeable to gas, but impervious to particles of the powder or granulate components filled into the cylinder (22) and/or to the paste-like mass prepared from the components.

10. Device according to claim 9, **characterized in that** the circular disk (50) made of filter material is held on the cylindrical end face of the piston (30) by a clip ring (58) having a larger diameter and extending over the outer edge of the circular disk (50) and/or by a clip ring (60) having a small diameter and extending over the inner edge of the circular disk (50).

11. Device according to claim 10, **characterized in that** the clip ring (58) having the larger diameter includes an attachment section that grips over a circumferential section (62) of the piston (30) with a diameter that is smaller than the unobstructed inside diameter of the cylinder (22), and/or the clip ring (60) having the small diameter includes an attachment section that engages with a bore section (64) having a diameter that is larger than the diameter of the through-opening (44) provided in the piston (30) for the mixing shaft (34).

12. Device according to claim 10 or 11, **characterized in that** the attachment section of the clip ring (58) gripping over the circumferential section (62) of the piston (30) with the smaller diameter and/or the attachment section of the clip ring (60) engaging with the bore section (64) in the piston (30) having the larger diameter is dimensioned in the direction of the longitudinal center axis of the piston (30) so as to be shorter than the corresponding circumferential or bore section of the piston, and that a corresponding sealing ring (54 and 56, respectively) is disposed in the groove-shaped recesses formed between the clip ring attachment sections and the ring-shaped surfaces formed in the piston in a region where the diameter of the circumferential section or bore section (62 and 64, respectively) changes.

13. Device according to claim 11 or 12, **characterized in that** the attachment section of the respective clip ring (58; 60), which grips over the circumferential section (62) of the piston and/or engages with the bore section (64), interlocks on or in the associated section of the piston (30), and/or is held thereon or therein with an adhesive and/or by a press fit.

## Revendications

1. Dispositif (20) pour préparer et appliquer un matériau, à préparer à partir d'au moins un composant sous forme de poudre ou de granulés et d'un composant liquide, directement avant utilisation, en une masse pâteuse coulante, à durcissement ou à prise différé(e), notamment du ciment à os, doté d'un cylindre mélangeur (22) allongé, prévu pour recevoir les composants à mélanger, lequel cylindre est muni à une extrémité d'une ouverture de passage (26) pouvant être obturée et facultativement d'un organe de distribution pour le matériau pâteux préparé, et d'un piston (30) pouvant être inséré dans l'autre extrémité opposée ouverte, mobile dans le cylindre (22) et étanchéifiant la paroi intérieure du cylindre, lequel dispositif est traversé de manière mobile dans le piston (30) par une tige mélangeuse (34) allongée étanche, un organe mélangeur (36) étant disposé à l'extrémité intérieure du cylindre de cette tige et une poignée (38) à l'extrémité extérieure du cylindre de cette tige, l'extrémité du cylindre (22) ouverte, opposée à l'ouverture de passage (26), logeant le piston (30) étant munie en outre d'un capuchon de fermeture (32) pouvant être fixé sur le cylindre ou le piston, et pouvant en être retiré, avec une ouverture de traversée (72) pour la tige mélangeuse (34), et le piston présente du côté du bouchon de fermeture un raccord d'aspiration (48) pour raccordement à une source de dépression, laquelle peut créer un vide à l'intérieur du cylindre à l'aide d'un passage dans le piston, une ouverture de passage (74) étant prévue dans le capuchon de fermeture, grâce à laquelle la conduite de raccordement menant à la source de dépression peut être raccordée au raccord d'aspiration dans le piston (30),
**caractérisé en ce que**
le capuchon de fermeture (32) pouvant être posé sur le bord de l'extrémité ouverte du cylindre (22) est relié de manière mobile à deux languettes de verrouillage (78) plates, parallèles l'une à l'autre situées dans un plan, avec ses côtés plats, lesquelles languettes sont prémontées dans respectivement une ouverture de passage (84) du piston (30) complémentaire à la section des languettes de verrouillage (78), traversant l'un des pistons (30) perpendiculairement à son axe médian longitudinal,
des évidements (86) ouverts débouchant sur le bord de l'extrémité ouverte du cylindre (22) et dotés d'une embouchure rétrécie pour loger les languettes de verrouillage (78) sont prévus, et
les languettes de verrouillage peuvent être pressées en sens transversal de manière élastique dans la zone située au niveau de l'extrémité ouverte du cylindre dans la position de verrouillage du piston (30) conforme aux dispositions, et donc peuvent être guidées dans les évidements (86) dans l'embouchure rétrécie des évidements (86) dans le cylindre (22), et maintiennent le capuchon de fermeture (32) et le cylindre (22) sur l'extrémité ouverte du cylindre (22).

2. Dispositif selon la revendication 1, **caractérisé en ce que** les languettes de verrouillage (78) sont articulées de manière pivotante sur le capuchon de fermeture (32).

3. Dispositif selon la revendication 2, **caractérisé en ce que** le capuchon de fermeture (32) et la (les) languette(s) de fermeture sont des pièces en plastique moulées par injection.

4. Dispositif selon la revendication 3, **caractérisé en ce que** les languettes de verrouillage (78) sont intégralement articulées sur le capuchon de fermeture (32) à l'aide d'une charnière intégrale (80).

5. Dispositif selon l'une quelconque des revendications 1 à 4, **caractérisé en ce que** les languettes de verrouillage (78) sont munies d'évidements (82) oblongs dans la zone située dans la position de verrouillage conforme aux dispositions dans les évidements (86) du cylindre (82), lesquels évidements permettent une déformation des zones de bord restantes les unes sur les autres.

6. Dispositif selon l'une quelconque des revendications 1 à 5, **caractérisé en ce que** le piston (30), la tige mélangeuse (34) traversant de manière mobile le piston (30), munie de l'organe mélangeur (36) à l'extrémité intérieure du cylindre et de la poignée (38) à l'extrémité extérieure, ainsi que le capuchon de fermeture (32) traversé par la tige mélangeuse (34) avec les languettes de verrouillage (78) prémontées dans les ouvertures de passage (84) dans le piston (30), sont insérés dans l'extrémité ouverte du cylindre (22) et prémontés dans l'un des modules encliquetables dans les évidements (86) prévus sur le bord de l'extrémité ouverte du cylindre (22) au moyen des languettes de verrouillage (78).

7. Dispositif selon la revendication 6, **caractérisé en ce que** la tige mélangeuse (34), lorsque l'organe mélangeur (36) est poussé entièrement jusqu'à buter sur la surface frontale à l'intérieur du cylindre du piston (30), est déjà munie dans la partie située dans la zone de la surface frontale extérieure du piston (30) détournée du cylindre, d'un tel point destiné à la rupture (66), le long duquel la pièce de la tige mélangeuse (34) à l'extérieur du piston (30) peut être brisée.

8. Dispositif selon l'une quelconque des revendications 1 à 7, **caractérisé en ce que** la tige mélangeuse (34) est formée par un petit tube alésé sur toute sa longueur, et **en ce que** le point destiné à la rupture (66) est formé par un creux entaillé, prévu à l'intérieur de la paroi du petit tube.

9. Dispositif selon la revendication 8, **caractérisé en ce que** le raccord d'aspiration dans le piston (30) est formé par une buse d'aspiration (48) dont la conduite d'aspiration (46) dans le piston (30) est guidée dans un creux (50) périphérique annulaire dans le côté frontal du côté du cylindre du piston (30), et **en ce que** le creux (50) périphérique annulaire est obturé vers l'intérieur du cylindre par un disque (52) poreux annulaire en matériau filtrant, dont la taille moyenne des pores est sélectionnée de telle sorte qu'il est perméable aux gaz, mais ne laisse pas passer des particules du composant poudreux ou granuleux rempli dans le cylindre (22) ni/ou la masse pâteuse préparée à partir des composants.

10. Dispositif selon la revendication 9, **caractérisé en ce que** le disque (50) annulaire en matériau filtrant est maintenu sur la surface frontale cylindrique du piston (30) par une bague de fixation (58) de diamètre supérieur chevauchant l'un de ses bords extérieurs et/ou par une bague de fixation (60) de diamètre inférieur chevauchant l'un de ses bords intérieurs.

11. Dispositif selon la revendication 10, **caractérisé en ce que** la bague de fixation (58) de plus grand diamètre présente une section de fixation ayant prise sur une section périphérique (62) du piston (30) de diamètre rétréci par rapport au diamètre intérieur du cylindre (22) et/ou la bague de fixation (60) de plus petit diamètre présente une section de fixation ayant prise dans une section d'alésage (64) de diamètre plus grand par rapport au diamètre de l'alésage de passage (44) de la tige mélangeuse (34), prévu dans le piston (30).

12. Dispositif selon la revendication 10 ou 11, **caractérisé en ce que** la section de fixation de la bague de fixation (58) de plus grand diamètre chevauchant la section périphérique (62) de diamètre réduit du piston, et/ou la section de fixation de la bague de fixation (60) de plus petit diamètre ayant prise dans la section d'alésage (64) de plus grand diamètre dans le piston (30), est moins longue, dans le sens de l'axe longitudinal médian du piston (30), que la section périphérique ou d'alésage associée du piston, et un joint d'étanchéité (54 ou 56) est disposé dans les creux en forme de rainure formés dans les surfaces annulaires existantes entre les sections de fixation de la bague de fixation et les surfaces annulaires existantes dans le piston dans la zone des modifications de diamètre de la section périphérique ou d'alésage (62 ou 64).

13. Dispositif selon la revendication 11 ou 12, **caractérisé en ce que** la section de fixation de la bague de fixation (58 ; 60) ayant prise sur la section périphérique du piston (62) et/ou dans la section d'alésage (64) est/sont encliquetée(s) et/ou collée(s) et/ou maintenue(s) sur l'ajustage serré dans la section associée du piston (30).
